# EUROPÄISCHE PATENTSCHRIFT

(11) **EP 0 997 192 B1**
(45) Veröffentlichungstag und Bekanntmachung des Hinweises auf die Patenterteilung: **24.10.2001**
(21) Anmeldenummer: 99116463.3
(22) Anmeldetag: 21.08.1999
(51) Int. Cl.: B01J 23/58, B01J 23/52, B01J 23/60, B01J 23/66, C07C 67/055, C07C 69/15

(54) **Trägerkatalysator für die Produktion von Vinylacetatmonomer**
Supported catalyst for the production of vinyl acetate monomer
Catalyseur supporté pour la production du monomère d'acétate de vinyle

(30) Priorität: 24.09.1998 DE 19843693
(43) Veröffentlichungstag der Anmeldung: 03.05.2000
(73) Patentinhaber: Degussa AG, 40474 Düsseldorf (DE)
(72) Erfinder: Tacke, Thomas, Dr., Paducah, KY 42001 (US); Krause, Helmfried, 63517 Rodenbach (DE); Lansink-Rotgerink, Hermanus, G.J., Dr., 63864 Glattbach (DE); Daly, Frank, Dr., 42071 Murray, KY (US); Reisinger, Martin, Dr., 63584 Haingründau (DE); Mangold, Helmut, Dr., 63517 Rodenbach (DE)

(56) Entgegenhaltungen:
- EP-A- 0 330 853
- EP-A- 0 403 950
- EP-A- 0 519 435
- EP-A- 0 723 810
- EP-A- 0 807 615
- US-A- 5 672 734

## Beschreibung

Die Erfindung betrifft einen Trägerkatalysator auf Basis von pyrogen hergestelltem Mischoxid, der sich insbesondere für die Produktion von Vinylacetatmonomer (VAM) eignet.

Pyrogen hergestellte Oxide zeichnen sich durch extreme Feinteiligkeit und entsprechend hohe spezifische Oberfläche, sehr hohe Reinheit, sphärische Teilchenform und das Fehlen von Poren aus. Aufgrund dieser Eigenschaften finden die pyrogen hergestellten Oxide zunehmend Interesse als Träger für Katalysatoren (D. Koth, H. Ferch, Chem. Ing. Techn. 52, 628 (1980)). In einigen Fällen werden pyrogene Oxide auch als Katalysator eingesetzt.

Da pyrogen hergestellte Oxide besonders feinteilig sind, bereitet die Verformung zu Katalysatorträgern beziehungsweise Katalysatoren einige Schwierigkeiten.

Aus der DE-B 21 00 778 ist es bekannt, Granulate auf Basis pyrogen hergestellter Siliciumdioxide als Katalysatorträger bei der Herstellung von Vinylacetatmonomer einzusetzen.

Weitere Produktionsverfahren zur Herstellung von Vinylacetatmonomer sind aus den Dokumenten DE 16 68 088, US 4,048,096, EP-A 0 519 435, EP-A 0 634 208, EP-A 0 723 810, EP-A 0 634 209, EP-A 0 632 214, EP-A 0 654 301 und EP-A 0 723 810 bekannt. Diese Dokumente beschreiben auch Verfahren zur Herstellung von Trägerkatalysatoren. Je nach Ausführungsform werden Trägerkatalysatoren mit homogener Edelmetallverteilung über den Trägerquerschnitt und mit mehr oder weniger ausgeprägtem Schalenprofil erhalten.

Die EP-A 0 723 810 beschreibt eine Vorbehandlung von Katalysatorträgern fuer die Herstellung von Vinylacetatmonomer mit Elementen der Gruppen IA, IIA, IIIA und IVB des Periodensystems.

Nachteilig ist bei diesen Verfahren aus dem Stand der Technik, daß diese zu Katalysatoren führen, die zu einem ungünstigen Verhältnis aus Selektivität und Aktivität bei der Herstellung von Vinylacetatmonomer führen.

Es ist bekannt, pyrogene Mischoxide herzustellen, indem man mindestens zwei unterschiedliche Metalle in Form flüchtiger Metallverbindungen, z.B. Chloride, in einer H₂/O₂ Flamme gleichzeitig umsetzt. Ein Beispiel hierfür ist das SiO₂/Al₂O₃-Mischoxid, welches von der Firma Degussa mit der Bezeichnung Aerosil® MOX 170 hergestellt und vertrieben wird. Bei der Herstellung des Aerosil® MOX 170 wird eine Mischung aus SiCl₄ und AlCl₃ direkt in einer Flamme hydrolysiert. Anstelle von oder zusätzlich zu den Chloriden können auch entsprechende Silane, wie z.B. Methyltrichlorsilan, Trichlorsilane, usw. als Rohstoffverwendet werden. (Degussa Technical Bulletin Pigments, No. 11: Basic Characteristics of Aerosil® , S. 37 sowie 11-12; AT-A 195 893; DE-A 952 891; DE-A 25 33 925; DE-A 27 02 896).

Die vorliegende Erfindung verwendet neue Formkörper auf Basis von pyrogen hergestelltem Mischoxid mit den folgenden physikalisch-chemischen Kenndaten:

| | |
|---|---|
| Außendurchmesser | 0,8 - 25 mm |
| BET-Oberfläche | 5 - 400 m²/g |
| Porenvolumen | 0,2 - 1,8 ml/g |
| Bruchfestigkeit | 5 bis 350 N |
| Zusammensetzung | Mindestens zwei aus der Gruppe SiO₂, Al₂O₃, TiO₂ und ZrO₂ in jeder beliebigen Kombination jedoch mit Ausnahme von SiO₂-Al₂O₃ Mischoxiden, |
| | |
| Sonstige Bestandteile | < 1 Gew.-% |
| Schüttgewicht | 250 - 1500 g/l |

Die erfindungsgemäßen Formkörper können als Strangpreßlinge, Extrudate oder Tabletten vorliegen. Sie können in Form von Zylindern, Zylindern mit abgerundeten Stirnflächen, Kugeln, Ringen, Wagenrädern, Minilithen oder in anderen für Festbettkatalysatoren üblichen Formen vorliegen.

Die Herstellung der erfindungsgemäßen Formkörper auf Basis von pyrogen hergestelltem Mischoxid mit folgenden physikalisch-chemischen Kenndaten:

| | |
|---|---|
| Außendurchmesser | 0,8 - 25 mm |
| BET-Oberfläche | 5 - 400 m²/g |
| Porenvolumen | 0,2 - 1,8 ml/g |
| Bruchfestigkeit | 5 - 350 N |
| Zusammensetzung | Mindestens zwei aus der Gruppe SiO₂, Al₂O₃, TiO₂ und ZrO₂ in jeder beliebigen Kombination jedoch mit Ausnahme von SiO₂-Al₂O₃ Mischoxiden, |
| | |
| Sonstige Bestandteile | < 1 Gew.-% |
| Schüttgewicht | 250 - 1500 g/l, |

kann erfolgen, indem man pyrogen hergestelltes Mischoxid wahlweise mit einer oder mehreren Verbindungen der Gruppe Methylcellulose, Methylhydroxyethylcellulose, Wachs, Magnesiumstearat, Aluminiumstearat und/oder Polyethylenglycol unter Zusatz von Wasser homogenisiert, bei einer Temperatur von 70 - 150 °C trocknet, gegebenenfalls zu einem Pulver zerkleinert, das Pulver zu Formkörpern verpreßt und während eines Zeitraumes von 0,5 bis 10 Stunden bei einer Temperatur von 400 bis 1200 °C tempert.

Die Herstellung der erfindungsgemäßen Formkörper kann auf Stempelpressen, Exzenterpressen, isostatischen Pressen, Strangpressen, Rundlaufpressen oder Kompaktoren erfolgen.

Vor dem Verpressen kann eine besondere Ausführungsform der Erfindung die folgende Zusammensetzung aufweisen:

| | |
|---|---|
| 50 - 90 Gew.-% | Mischoxid |
| 0,1 - 20 Gew.-% | Methylhydroxyethylcellulose, vorzugsweise 5 - 15 Gew.-% |
| 0,1 - 15 % | Wachs, vorzugsweise 5 - 12 Gew.-% |
| 0,1 - 15 % | Polyethylenglykol, vorzugsweise 5 - 10 Gew.-% |

Durch Variation der Einsatzstoffmengen und des Preßdruckes können die Bruchfestigkeit, die spezifische Gesamtoberfläche und das Porenvolumen in einem gewissen Rahmen eingestellt werden.

Alternativ kann die Herstellung der erfindungsgemäßen Formkörper auf Basis von pyrogen hergestelltem Mischoxid mit folgenden physikalisch-chemischen Kenndaten:

| | |
|---|---|
| Außendurchmesser | 0,8 - 25 mm |
| BET-Oberfläche | 5 - 400 m²/g |
| Porenvolumen | 0,2 - 1,8 ml/g |
| Bruchfestigkeit | 5 - 350 N |
| Zusammensetzung | Mindestens zwei aus der Gruppe SiO₂, Al₂O₃, TiO₂ und ZrO₂ in jeder beliebigen Kombination jedoch mit Ausnahme von SiO₂-Al₂O₃ Mischoxiden |
| | |
| Sonstige Bestandteile | < 1 Gew.-% |
| Schüttgewicht | 250 - 1500 g/l |

erfolgen, indem man pyrogen hergestelltes Mischoxid wahlweise mit einer oder mehreren Verbindungen der Gruppe Methylcellulose, Methylhydroxyethylcellulose, Wachs, Magnesiumstearat, Aluminiumstearat und/oder Polyethylenglycol unter Zusatz von Wasser homogenisiert, einem Knet- und Formgebungsverfahren unterzieht, extrudiert, die Extrudate gegebenfalls mittels einer Schneidevorrichtung auf die gewünschte Länge schneidet, bei einer Temperatur von 70 - 150 °C trocknet und während eines Zeitraumes von 0,5 bis 10 Stunden bei einer Temperatur von 400 bis 1200 °C tempert.

Zur Durchführung der erfindungsgemäßen Verfahren sind alle Mischer oder Mühlen, die eine gute Homogenisierung ermöglichen, wie zum Beispiel Schaufel-, Wirbelschicht-, Kreisel- oder Luftstrommischer, geeignet. Besonders geeignet sind Mischer, mit denen eine zusätzliche Verdichtung des Mischgutes möglich ist, wie zum Beispiel Pflugscharmischer, Kollergänge oder Kugelmühlen. Die Mischung und Knetung kann aber auch direkt in einem Extruder erfolgen. Die Herstellung der Extrudate kann auf Ein- oder Zweischneckenextrudern, Strangpressen als auch auf Kompaktoren erfolgen.

Nach dem Homogenisieren kann eine weitgehende Trocknung bei 70 - 150 °C erfolgen, so daß man nach gegebenenfalls durchgeführtem Zerkleinern ein rieselfähiges Pulver erhält.

Die erfindungsgemäßen Formkörper können entweder direkt als Katalysator oder als Katalysatorträger eingesetzt werden.

Eine Aufgabe der vorliegenden Erfindung ist es, einen Trägerkatalysator anzugeben, welcher bei gleicher beziehungsweise verbesserter Selektivität eine höhere Aktivität als bekannte Katalysatoren aufweist.

Ein Gegenstand der Erfindung ist daher ein Trägerkatalysator, der auf einem Träger als katalytisch aktive Komoponenten Palladium und/oder dessen Verbindungen und Alkaliverbindungen, sowie zusätzlich Gold und/oder dessen Verbindungen (System Pd/Alkali/Au) oder Cadmium und/oder dessen Verbindungen (System Pd/Alkali/Cd) oder Barium und/oder dessen Verbindungen (System Pd/Alkali/Ba) oder Palladium, Alkaliverbindungen und Mischungen aus Gold und/oder Cadmium und/oder Barium enthält dadurch gekennzeichnet, daß es sich bei dem Träger im einen Formkörper auf Basis von pyrogen hergestelltem Mischoxid mit folgenden physikalisch-chemischen Kenndaten handelt:

| | |
|---|---|
| Außendurchmesser | 0,8 - 25 mm |
| BET-Oberfläche | 5 - 400 m²/g |
| Porenvolumen | 0,2 - 1,8 ml/g |
| Bruchfestigkeit | 5 - 350 N |
| Zusammensetzung | Mindestens zwei aus der Gruppe SiO₂, Al₂O₃, TiO₂ und ZrO₂ in jeder beliebigen Kombination jedoch mit Ausnahme von SiO₂-Al₂O₃ Mischoxiden |
| | |
| Sonstige Bestandteile | < 1 Gew.-% |
| Schüttgewicht | 250 - 1500 g/l |

Als Alkaliverbindungen sind Kaliumverbindungen, wie z. B. Kaliumacetat, bevorzugt.

Die katalytisch aktiven Komponenten können in folgenden Systemen vorhanden sein:
Pd/Au/Alkali-Verbindungen
Pd/Cd/Alkali-Verbindungen
Pd/Ba/Alkali-Verbindungen

Die erfindungsgemäßen Trägerkatalysatoren werden insbesondere für die Produktion von Vinylacetatmonomer verwendet. Dazu werden Ethen, Essigsäure und molekularer Sauerstoff bzw. Luft in der Gasphase, gegebenenfalls unter Zusatz von Inertgasen, bei Temperaturen zwischen 100 und 250 °C und bei normalem oder erhöhtem Druck in Gegenwart des erfindungsgemäßen Trägerkatalysators zur Reaktion gebracht. Grundsätzlich können diese Katalysatoren jedoch auch zur Acetoxylierung von Olefinen, wie beispielsweise Propen, eingesetzt werden.

Ein weiterer Gegenstand der Erfindung ist ein Verfahren zur Herstellung des erfindungsgemäßen Trägerkatalysators durch Tränken, Aufsprühen, Aufdampfen, Tauchen oder Ausfällen der Pd-, Au, Cd-, Ba-Metallverbindungen, gegebenenfalls Reduzieren der auf dem Träger aufgebrachten reduzierbaren Metallverbindungen, Waschen zur Entfernung gegebenenfalls vorhandener Chloridanteile, Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, in geeigneter Reihenfolge, wobei der Träger ein Formkörper auf Basis von pyrogen hergestelltem Mischoxid ist.

Ein weiterer Gegenstand der Erfindung ist ein Verfahren zur Herstellung des erfindungsgemäßen Trägerkatalysators durch Imprägnieren des Trägers mit einer basischen Lösung und einer Gold- und Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, wobei der Träger ein Formkörper auf Basis von pyrogen hergestelltem Mischoxid ist.

Die erfindungsgemäßen Trägerkatalysatoren können zur Herstellung ungesättigter Ester aus Olefinen, Säuren und Sauerstoff in der Gasphase eingesetzt werden.

Die erfindungsgemäßen Katalysatoren des Katalysatorsystems Pd/Alkali/Au können durch Imprägnieren der Träger mit einer basischen Lösung und einer Gold- und Palladiumsalze enthaltenden Lösung erhalten werden, wobei die Imprägnierschritte gleichzeitig oder nacheinander, mit oder ohne Zwischentrocknung erfolgen können. Anschließend kann der Träger zur Entfernung gegebenenfalls vorhandener Chloridanteile gewaschen werden. Vor oder nach dem Waschen können die auf dem Träger ausgefällten unlöslichen Edelmetallverbindungen reduziert werden. Die so erhaltene Katalysatorvorstufe kann getrocknet und zur Aktivierung des Katalysators mit Alkaliacetaten oder Alkaliverbindungen, die sich unter den Reaktionsbedingungen bei der Produktion von Vinylacetatmonomer ganz oder teilweise in Alkaliacetate umwandeln, imprägniert werden. Im allgemeinen können die Edelmetalle bei Pd/Au-Katalysatoren in Form einer Schale auf dem Träger vorliegen.

Bei Pd/Alkali/Ba-Katalysatoren können die Metallsalze durch Tränken, Aufsprühen, Aufdampfen, Tauchen oder Ausfällen aufgebracht werden (EP 0 519 436). Dieselben Methoden sind bei Pd/Alkali/Cd-Katalysatoren bekannt (US-PS 4,902,823, US-PS 3,393,199, US-PS 4,668,819).

Je nach Katalysatorsystem kann eine Reduktion des Trägerkatalysators vorgenommen werden.

Die Reduktion des Katalysators kann in der wäßrigen Phase oder in der Gasphase vorgenommen werden. Zur Reduktion in der wäßrigen Phase eignen sich zum Beispiel Formaldehyd oder Hydrazin. Die Reduktion in der Gasphase kann mit Wasserstoff, beziehungsweise Formiergas (95 Vol.-% N₂ + 5 Vol.-% H₂), Ethen oder stickstoffverdünntem Ethen vorgenommen werden. Gemäß der EP 0 634 209 erfolgt die Reduktion mit Wasserstoff bei Temperaturen zwischen 40 und 260 °C, bevorzugt zwischen 70 und 200 °C. Gemäß der EP-A 0 723 810 erfolgt die Reduktion mit Formiergas (95 Vol.-% N₂ und 5 Vol.-% H₂) bei Temperaturen zwischen 300 und 550 °C, bevorzugt zwischen 350 und 500 °C. Häufig wird der Katalysator jedoch erst nach der Aktivierung mit Alkaliacetat direkt im Produktionsreaktor mit Ethen reduziert.

Im Produktionsprozeß wird der Katalysator erst langsam mit den Reaktanden belastet. Während dieser Anfahrphase erhöht sich die Aktivität des Katalysators und erreicht gewöhnlich erst nach Tagen oder Wochen ihr endgültiges Niveau.

Wichtig ist, daß die Katalysatorträger unter den Reaktionsbedingungen des katalytischen Prozesses, insbesondere unter dem Einfluß von Essigsäure, ihre mechanische Festigkeit behalten.

Der erfindungsgemäße Trägerkatalysator erreicht bei hoher Aktivität hervorragende Selektivitäten.

Im folgenden wird die Herstellung von Trägerkatalysatoren des Systems Pd/Alkali/Au auf dem erfindungsgemäßen Träger (Formkörper) näher beschrieben.

Die erfindungsgemäßen Formkörper auf Basis von pyrogen hergestelltem Mischoxid können mit einer Palladium und Gold enthaltenden Lösung imprägniert werden. Gleichzeitig mit der edelmetallhaltigen Lösung oder in beliebiger Reihenfolge nacheinander können die erfindungsgemäßen Formkörper mit einer basischen Lösung, welche ein oder mehrere basische Verbindungen enthalten kann, imprägniert werden. Die basische Verbindung oder Verbindungen dienen zur Überführung des Palladiums und Golds in ihre Hydroxide.

Die Verbindungen in der basischen Lösung können aus Alkalihydroxiden, Alkalibicarbonaten, Alkalicarbonaten, Alkalisilikaten oder Mischungen dieser Stoffe bestehen. Bevorzugt können Kaliumhydroxid und/oder Natriumhydroxid verwendet werden.

Zur Herstellung der edelmetallhaltigen Lösung können als Palladiümsalze, beispielsweise Palladiumchlorid, Natriumbeziehungsweise Kaliumpalladiumchlorid oder Palladiumnitrat, verwendet werden. Als Goldsalze eignen sich Gold(III)-chlorid und Tetrachlorogold(III)-säure. Vorzugsweise können Kaliumpalladiumchlorid, Natriumpalladiumchlorid und/oder Tetrachlorogoldsäure eingesetzt werden.

Das Imprägnieren der erfindungsgemäßen Formkörper mit der basischen Lösung beeinflußt die Abscheidung der Edelmetalle in dem Formkörper. Die basische Lösung kann entweder gleichzeitig mit der Edelmetallösung oder in beliebiger Reihenfolge mit dieser Lösung mit dem erfindungsgemäßen Formkörper in Kontakt gebracht werden. Bei aufeinanderfolgender Imprägnierung des erfindungsgemäßen Formkörpers mit den beiden Lösungen kann nach dem ersten Imprägnierschritt eine Zwischentrocknung vorgenommen werden.

Bevorzugt kann der erfindungsgemäße Formkörper zuerst mit der basischen Verbindung imprägniert werden. Die anschließende Imprägnierung mit der Palladium und Gold enthaltenden Lösung kann zur Ausfällung von Palladium und Gold in einer oberflächlichen Schale auf dem erfindungsgemäßen Formkörper führen. Die umgekehrte Reihenfolge der Imprägnierungen kann im allgemeinen zu einer mehr oder weniger homogenen Verteilung der Edelmetalle über den Querschnitt des erfindungsgemäßen Formkörpers führen. Bei geeigneter Verfahrensführung können jedoch auch bei umgekehrter Imprägnier-Reihenfolge Katalysatoren mit definierter Schale erhalten werden (siehe z. B. US 4,048,096). Katalysatoren mit homogener oder nahezu homogener Edelmetall-Verteilung können im allgemeinen eine geringere Aktivität und Selektivität aufweisen.

Träger-Katalysatoren mit Schalendicken unter 1 mm, bevorzugt unter 0,8 mm, sind besonders geeignet. Die Schalendicke kann durch die Menge der auf das Trägermaterial aufgebrachten basischen Verbindung relativ zur gewünschten Menge der Edelmetalle beeinflußt werden. Je höher dieses Verhältnis ist, desto geringer wird die Dicke der sich ausbildenden Schale. Das für eine gewünschte Schalendicke erforderliche Mengenverhältnis von basischer Verbindung zu den Edelmetallverbindungen kann von der Beschaffenheit des Trägermaterials sowie von der gewählten basischen Verbindung und den Edelmetallverbindungen abhängen. Das erforderliche Mengenverhältnis wird zweckmäßigerweise durch wenige Vorversuche ermittelt. Die sich ergebende Schalendicke kann dabei in einfacher Weise durch aufschneiden der Katalysatorteilchen ermittelt werden.

Die minimal notwendige Menge der basischen Verbindung ergibt sich aus der stöchiometrisch berechneten Menge an Hydroxidionen, die zur Überführung des Palladiums und Golds in die Hydroxide benötigt werden. Als Richtwert gilt, daß die basische Verbindung für eine Schalendicke von 0,5 mm in einem 1 bis 10-fachen stöchiometrischen Überschuß angewendet werden sollte.

Die erfindungsgemäßen Formkörper können nach dem Verfahren der Porenvolumenimprägnierung mit den basischen Verbindungen und den Edelmetallsalzen belegt werden. Wird mit Zwischentrocknung gearbeitet, wählt man die Volumina der beiden Lösungen so, daß sie jeweils etwa 90 bis 100 % der Aufnahmekapazität der erfindungsgemäßen Formkörper entsprechen. Wird auf die Zwischentrocknung verzichtet, so muß die Summe der Einzelvolumina der beiden Imprägnierlösungen der obigen Bedingung entsprechen, wobei die Einzelvolumina im Verhältnis von 1 : 9 bis 9 : 1 zueinander stehen können. Bevorzugt wird ein Volumenverhältnis von 3 : 7 bis 7 : 3, insbesondere von 1 : 1, angewendet. Als Lösungsmittel kann in beiden Fällen bevorzugt Wasser verwendet werden. Es können aber auch geeignete organische oder wäßrig-organische Lösungsmittel eingesetzt werden.

Die Umsetzung der Edelmetallsalzlösung mit der basischen Lösung zu unlöslichen Edelmetallverbindungen kann langsam erfolgen und ist je nach Präparationsmethode im allgemeinen erst nach 1 bis 24 Stunden abgeschlossen. Danach werden die wasserunlöslichen Edelmetallverbindungen mit Reduktionsmitteln behandelt. Es kann eine Naßreduktion, zum Beispiel mit wäßrigem Hydrazinhydrat, oder eine Gasphasenreduktion mit Wasserstoff, Ethen, Formiergas oder Methanoldämpfen vorgenommen werden. Die Reduktion kann bei Normaltemperatur oder erhöhter Temperatur und bei Normaldruck oder erhöhtem Druck, gegebenenfalls auch unter Zugabe von Inertgasen, erfolgen.

Vor und/oder nach der Reduktion der Edelmetallverbindungen kann das auf dem Träger gegebenenfalls vorhandene Chlorid durch eine gründliche Waschung entfernt werden. Nach der Waschung kann der Katalysator weniger als 500, vorzugsweise weniger als 200 ppm, Chlorid enthalten.

Die nach der Reduktion erhaltene Katalysatorvorstufe kann getrocknet und abschließend mit Alkaliacetaten oder Alkaliverbindungen, die sich unter den. Reaktionsbedingungen bei der Produktion von Vinylacetatmonomer ganz oder teilweise in Alkaliacetate umwandeln, imprägniert werden. Vorzugsweise kann mit Kaliumacetat imprägniert werden. Hierbei kann wieder bevorzugt die Porenvolumenimprägnierung angewandt werden. Das heißt: Die benötigte Menge an Kaliumacetat wird in einem Lösungsmittel, vorzugsweise Wasser, dessen Volumen etwa der Aufnahmekapazität des vorgelegten Trägermaterials für das gewählte Lösungsmittel entspricht, gelöst. Dieses Volumen ist etwa gleich dem Gesamtporenvolumen des Trägermaterials.

Der fertige Katalysator kann anschießend bis auf eine Restfeuchte von weniger als 5 % getrocknet werden. Die Trocknung kann an der Luft, gegebenenfalls auch unter Stickstoff als Inertgas, erfolgen.

Die Herstellung von Trägerkatalysatoren der Systeme Pd/Alkali/Cd bzw. Pd/Alkali/Ba auf den erfindungsgemäßen Formkörpern kann nach den oben zitierten Patentschriften erfolgen.

Für die Synthese von Vinylacetatmonomer ist es zweckmäßig, den Katalysator mit 0,2 bis 4, vorzugsweise 0,3 bis 3 Gew.-% Palladium, 0,1 bis 2, vorzugsweise 0,15 bis 1,5 Gew.-% Gold und 1 bis 10, vorzugsweise 1,5 bis 9 Gew.-% Kaliumacetat, jeweils bezogen auf das Gewicht des eingesetzten Trägers, zu belegen. Diese Angaben gelten für das System Pd/Alkali/Au. Im Fall von Katalysatorträgern mit einer Schüttdichte von 500 g/l entsprechen diese Konzentrationsangaben volumenbezogenen Konzentrationen von 1,0 bis 20 g/l Palladium, 0,5 bis 10 g/l Gold und 5 bis 50 g/l Kaliumacetat. Zur Anfertigung der Imprägnierlösungen können die entsprechenden Mengen der Palladium- und Goldverbindungen in einem Volumen Wasser, welches etwa 10 bis 100 % der Wasseraufnahmekapazität des vorgelegten Trägermaterials entspricht, gelöst werden. Ebenso kann bei der Anfertigung der basischen Lösung verfahren werden.

Der Cadmium-Gehalt der Pd/Alkali/Cd-Katalysatoren kann 0,1 bis 2,5 Gew.-%, vorzugsweise 0,4 bis 2,0 Gew.-% betragen.

Der Barium-Gehalt der Pd/Alkali/Ba-Katalysatoren kann 0,1 bis 2,0 Gew.-%, vorzugsweise 0,2 bis 1,8 Gew.-% betragen.

Der Palladium-Gehalt der Pd/Alkali/Cd beziehungsweise Pd/Alkali/Ba-Katalysatoren kann 0,2 bis 4 Gew.-%, bevorzugt 0,3 bis 3 Gew.-% Palladium, betragen.

Der Kalium-Gehalt der Pd/Alkali/Cd- bzw. Pd/Alkali/Ba-Katalysatoren kann 1 bis 10 Gew.-%, vorzugsweise 1,5 bis 9 Gew.-% betragen.

Zur Herstellung von Mischoxiden wird in eine Knallgasflamme aus Wasserstoff und Luft flüchtige Metallverbindungen eingedüst. Diese Metallverbindungen hydrolysieren unter dem Einfluß des bei der Knallgasreaktion entstehenden Wassers zu Metalloxiden und Salzsäure. Das Metalloxid tritt nach dem Verlassen der Flamme in eine sogenannte Koagulationszone ein, in der die Mischoxid-Primärteilchen und -Primäraggregate agglomerieren. Das in diesem Stadium als eine Art Aerosol vorliegende Produkt wird in Zyklonen von den gasförmigen Begleitsubstanzen getrennt und anschließend mit feuchter Heißluft nachbehandelt. Durch dieses Verfahren läßt sich der Rest-Salzsäuregehalt unter 0,025 % senken. Da das Mischoxid am Ende dieses Prozesses mit einer Schüttdichte von nur ca. 15 g/l anfällt, kann eine Vakuumverdichtung, mit der sich Stampfdichten von ca. 50 g/l und mehr einstellen lassen, angeschlossen werden.

Die Teilchengrößen der auf diese Weise gewonnenen Produkte können mit Hilfe der Reaktionsbedingungen, wie zum Beispiel die Flammentemperatur, der Wasserstoff- oder Sauerstoffanteil, die Metallchloridsubstanzen und -menge, die Verweilzeit in der Flamme oder die Länge der Koagulationsstrecke, variiert werden.

Die BET-Oberfläche wird gemäß DIN 66 131 mit Stickstoff bestimmt. Das Porenvolumen wird rechnerisch aus der Summe von Mikro-, Meso- und Makroporenvolumen bestimmt. Die Bruchfestigkeit wird mittels des Bruchfestigkeitstesters der Firma Erweka, Typ TBH 28, bestimmt.

Die Bestimmung der Mikro- und Mesoporen erfolgt durch Aufnahme einer N₂-Isotherme und deren Auswertung nach BET, de Boer und Barret, Joyner, Halenda.

Das Schüttgewicht wird auf übliche und dem Fachmann bekannte Weise bestimmt.

Die Bestimmung der Makroporen erfolgt durch das Hg-Einpreßverfahren.

Die Beispiele 1 - 6 zeigen exemplarisch die Herstellung der erfindungsgemäßen Formkörper auf Basis von pyrogen hergestelltem Mischoxid.

### Beispiel 1

| | |
|---|---|
| 71.4 Gew.-% | pyrogenes SiO₂-TiO₂ Mischoxid (70 Gew.-% SiO₂, 30 Gew.-% TiO₂) |
| 12.9 Gew.-% | Methylhydroxyethylcellulose |
| 7.1 Gew.-% | Wachs |
| 8.6 Gew.-% | Polyethylenglycol |

werden unter Zusatz von Wasser kompaktiert, bei 90 °C getrocknet, zu einem rieselfähigen Pulver zerkleinert und mit einer Excenterpresse zu Formkörpern verformt. Die Rohtabletten werden 6 Stunden bei 750 °C kalziniert.

### Beispiel 2

| | |
|---|---|
| 71.4 Gew.-% | pyrogenes SiO₂-TiO₂ Mischoxid (82 Gew.-% SiO₂, 18 Gew.-% TiO₂) |
| 12.9 Gew.-% | Methylhydroxyethylcellulose |
| 7.1 Gew.-% | Wachs |
| 8.6 Gew.-% | Polyethylenglycol |

werden unter Zusatz von Wasser kompaktiert, bei 100 °C getrocknet, zu einem rieselfähigen Pulver zerkleinert und mit einer Excenterpresse zu Formkörpern verformt. Die Rohtabletten werden 6 Stunden bei 700 °C kalziniert.

### Beispiel 3

| | |
|---|---|
| 71.4 Gew.-% | pyrogenes SiO₂-TiO₂ Mischoxid (91 Gew.-% SiO₂, 9 Gew.-% TiO₂) |
| 12.9 Gew.-% | Methylhydroxyethylcellulose |
| 7.1 Gew.-% | Wachs |
| 8.6 Gew.-% | Polyethylenglycol |

werden unter Zusatz von Wasser kompaktiert, bei 100 °C getrocknet, zu einem rieselfähigen Pulver zerkleinert und mit einer Excenterpresse zu Formkörpern verformt. Die Rohtabletten werden 10 Stunden bei 600 °C kalziniert.

### Beispiel 4

| | |
|---|---|
| 71.4 Gew.-% | pyrogenes SiO₂-TiO₂ Mischoxid (91 Gew.-% SiO₂, 9 Gew.-% TiO₂) |
| 12.9 Gew.-% | Methylhydroxyethylcellulose |
| 7.1 Gew.-% | Wachs |
| 8.6 Gew.-% | Polyethylenglycol |

werden unter Zusatz von Wasser kompaktiert, bei 100 °C getrocknet, zu einem rieselfähigen Pulver zerkleinert und mit einer Excenterpresse zu Formkörpern verformt. Die Rohtabletten werden 6 Stunden bei 750 °C kalziniert.

### Beispiel 5

| | |
|---|---|
| 90.0 Gew.-% | pyrogenes TiO₂-ZrO₂ Mischoxid (94 Gew.-% TiO₂, 6 Gew.-% ZrO₂) |
| 5.0 Gew.-% | Methylhydroxyethylcellulose |
| 2.0 Gew.-% | Wachs |
| 3.0 Gew.-% | Polyethylenglycol |

werden unter Zusatz von Wasser kompaktiert, bei 100 °C getrocknet, zu einem rieselfähigen Pulver zerkleinert und mit einer Excenterpresse zu Formkörpern verformt. Die Rohtabletten werden 10 Stunden bei 400 °C kalziniert.

### Beispiel 6

| | |
|---|---|
| 92.6 Gew.-% | pyrogenes TiO₂-ZrO₂ Mischoxid (94 Gew.-% TiO₂, 6 Gew.-% ZrO₂) |
| 0.9 Gew.-% | Methylhydroxyethylcellulose |
| 6.5 Gew.-% | Wachs |

werden unter Zusatz von Wasser kompaktiert, bei 100 °C getrocknet, zu einem rieselfähigen Pulver zerkleinert und mit einer Excenterpresse zu Formkörpern verformt. Die Rohtabletten werden 10 Stunden bei 400 °C kalziniert.

Die erhaltenen Formkörper weisen folgende physikalischchemische Kenndaten auf:

| **Beispiel** | **1** | **2** | **3** | **4** | **5** | **6** |
|---|---|---|---|---|---|---|
| Tablettenform | Zylinder | Zylinder | Zylinder | Zylinder | Ringe | Ringe |
| Außendurchmesser x Höhe x Innendurchmesser (mm) | 5x5 | 6x5.5 | 6x5.5 | 5x5 | 8x5x3 | 8x5x3 |
| BET-Oberfläche (m²/g) | 86 | 101 | 209 | 191 | 44 | 46 |
| Porenvolumen (ml/g) | 0.69 | 0.59 | 0.83 | 0.72 | 0.34 | 0.42 |
| Bruchfestigkeit (N) | 21 | 34 | 37 | 66 | 15 | 12 |
| Schüttgewicht (g/l) | n.b. | 610 | 505 | n.b. | 860 | 800 |

In den folgenden Beispielen wird die Leistungsfähigkeit des erfindungsgemäßen Trägerkatalysators exemplarisch dargestellt.

### Beispiel 7 (Vergleichsbeispiel)

Es wurde ein Palladium-Gold-Kaliumacetat-Katalysator nach Beispiel 10 der EP 0 807 615 A1 hergestellt. Hierzu wurde auf einem Katalysatorträger aus pyrogener Kieselsäure (BET-Oberfläche 168 m²/g, Schüttdichte 470 g/l, gesamtes Porenvolumen 0.84 cm³/g, Tabletten von 6 mm Durchmesser und 5,5 mm Hoehe, Mg-Gehalt < 50 Mikrogramm/g) ein Palladium-Gold-Kaliumacetat-Katalysator hergestellt. Die Konzentration der Imprägnierlösungen wurde so gewählt, daß der fertige Katalysator eine Konzentration von 0.55 Gew.-% Palladium, 0.25 Gew.-% Gold und 5.0 Gew.-% Kaliumacetat enthielt.

In einem ersten Schritt wurde der Träger zunächst mit einer basischen Lösung aus Natriumhydroxid in Wasser imprägniert. Das Volumen der wäßrigen NaOH-Lösung entsprach 50 Prozent der Wasseraufnahme des trockenen Trägers. Nach der Imprägnierung mit Natriumhydroxid wurde der Träger unmittelbar ohne Zwischentrocknung mit einer wäßrigen Edelmetalllösung aus Natriumpalladiumchlorid und Tetrachlorogoldsäure imprägniert, deren Volumen ebenfalls 50 Prozent der Wasseraufnahmekapazität des trockenen Trägermaterials entsprach.

Nach einer Wartezeit von 1,5 Stunden zwecks Hydrolyse der Edelmetallverbindungen wurden die Trägerteilchen chloridfrei gewaschen. Der Katalysator wurde getrocknet und bei 450 °C in der Gasphase mit Formiergas reduziert. Danach wurde der Katalysator mit einer wäßrigen Kaliumacetat-Lösung imprägniert und erneut getrocknet. Die Trocknung wurde in der Gasphase mit Stickstoff durchgeführt.

Die Konzentration der basischen Lösung an Natriumhydroxid war so bemessen, daß sich auf den Trägerteilchen eine edelmetallhaltige Schale von < 1.0 mm ausbildete. Dies ist der nicht-erfindungsgemäße Katalysator A.

### Beispiel 8

Auf identische Art und Weise wie im Beispiel 7 (Vergleichsbeispiel) wurde ein Pd-Au-K-Katalysator auf Basis des Formkörpers gemäß Beispiel 1 hergestellt. Dieser Katalysator enthielt ebenfalls 0.55 Gew.-% Palladium, 0.25 Gew.-% Gold und 5.0 Gew.-% Kaliumacetat. Dies ist der erfindungsgemäße Katalysator B.

### Beispiel 9

Der nicht-erfindungsgemäße Katalysator A (Beispiel 7) und der erfindungsgemäße Katalysator B (Beispiel 8) wurden beide getestet für die Herstellung von Vinylacetatmonomer (VAM).

Aktivität und Selektivität der Katalysatoren wurden während einer Prüfung von bis zu 24 Stunden gemessen.

Die Katalysatoren wurden in einem mit Öl beheizten Strömungsrohrreaktor (Reaktorlänge 710 mm, Innendurchmesser 23,7 mm) bei Normaldruck und einer Raumgeschwindigkeit (GHSV) von 550 h⁻¹ mit der folgenden Gaszusammensetzung geprüft: 75 Vol.-% Ethen, 16,6 Vol.-% Essigsäure, 8,3 Vol.-% Sauerstoff. Die Katalysatoren wurden im Temperaturbereich von 120 bis 165°C, gemessen im Katalysatorbett, untersucht.

Die Reaktionsprodukte wurden im Ausgang des Reaktors mittels on-line Gaschromatographie analysiert. Als Maß für die Katalysatoraktivität wurde die Raum-Zeit-Ausbeute des Katalysators in Gramm Vinylacetat-Monomer pro Stunde und Liter Katalysator (g VAM/(h x l_{Kat.}) bestimmt.

Kohlendioxid, das insbesondere durch die Verbrennung von Ethen gebildet wird, wurde ebenfalls bestimmt und zur Beurteilung der Katalysatorselektivität herangezogen.

Die Testergebnisse sind in der untenstehenden Tabelle zusammengefaßt. Die Aktivität und Selektiviät werden als relative Größe ausgedrückt, die Aktivität und Selektivität des nicht-erfindungsgemäßen Katalysators wurden auf 100% gestellt. Wie die Daten eindeutig belegen, ist der erfindungsgemäße Katalysator B sowohl was Aktivität als auch was Selektivität betrifft deutlich besser als der Vergleichskatalysator A.

| Katalysator | Relative Aktivität auf Basis von g VAM/(h x l_{Kat.}) in [%]∗ | Relative Selektivität CO₂ im Abgas auf Basis von Flächen-% in [%] ∗ | Katalysatortemperatur [°C] |
|---|---|---|---|
| A (nicht-erfindungsgemäß) | 100 | 100 | 162,5 |
| B (erfindungsgemäß) | 111,2 | 82,7 | 164,2 |

| | | | |
|---|---|---|---|
| ∗: siehe Beschreibung für die Definition | | | |

## Patentansprüche

1. Trägerkatalysator, der auf einem Träger als katalytisch aktive Komoponenten Palladium und/oder dessen Verbindungen und Alkaliverbindungen, sowie zusätzlich Gold und/oder dessen Verbindungen (System Pd/Alkali/Au) oder Cadmium und/oder dessen Verbindungen (System Pd/Alkali/Cd) oder Barium und/oder dessen Verbindungen (System Pd/Alkali/Ba) oder Palladium, Alkaliverbindungen und Mischungen aus Gold und/oder Cadmium und/oder Barium enthält, **dadurch gekennzeichnet, daß** es sich bei dem Träger im einen Formkörper auf Basis von pyrogen hergestelltem Mischoxid mit folgenden physikalisch-chemischen Kenndaten handelt:
| | |
|---|---|
| Außendurchmesser | 0,8 - 25 mm |
| BET-Oberfläche | 5 - 400 m²/g |
| Porenvolumen | 0,2 - 1,8 ml/g |
| Bruchfestigkeit | 5 - 350 N |
| Zusammensetzung | Mindestens zwei aus der Gruppe SiO₂, Al₂O₃, TiO₂ und ZrO₂ in jeder beliebigen Kombination jedoch mit Ausnahme von SiO₂-Al₂O₃ Mischoxiden |
| | |
| Sonstige Bestandteile | < 1 Gew.-% |
| Schüttgewicht | 250 - 1500 g/l |

2. Trägerkatalysator gemäß Anspruch 1, **dadurch gekennzeichnet, daß** als aktive Komponenten das System Pd/K/Au verwendet wird.

3. Trägerkatalysator gemäß Anspruch 1 und/oder 2, **dadurch gekennzeichnet, daß** es sich bei der Alkaliverbindung um Kaliumacetat handelt.

4. Verfahren zur Herstellung des Trägerkatalysators nach einem oder mehreren der Ansprüche 1 bis 3 durch Tränken, Aufsprühen, Aufdampfen, Tauchen oder Ausfällen der Pd-, Au, Cd-, Ba-Metallverbindungen, gegebenenfalls Reduzieren der auf dem Träger aufgebrachten reduzierbaren Metallverbindungen, Waschen zur Entfernung gegebenenfalls vorhandener Chloridanteile, Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, in geeigneter Reihenfolge, **dadurch gekennzeichnet, dass** der Träger ein Formkörper nach Anspruch 1 ist.

5. Verfahren zur Herstellung des Trägerkatalysators nach einem oder mehreren der Ansprüche 1 bis 3 durch Imprägnieren des Trägers mit einer basischen Lösung und einer Gold- und Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, **dadurch gekennzeichnet, dass** der Träger ein Formkörper nach Anspruch 1 ist.

6. Verfahren nach Anspruch 4 oder 5, **dadurch gekennzeichnet, daß** es sich bei dem Alkaliacetat bzw. der Alkaliverbindung um Kaliumacetat handelt.

7. Verwendung eines Trägerkatalysators nach einem oder mehreren der Ansprüche 1 bis 3 zur Herstellung ungesättigter Ester aus Olefinen, organischen Säuren und Sauerstoff in der Gasphase.

8. Verwendung eines Trägerkatalysators gemäß Ansprüche 7 zur Herstellung von Vinylacetatmonomer.

## Claims

1. Supported catalyst which contains palladium and/or the compounds thereof and alkali metal compounds, as well as additionally gold and/or the compounds thereof (Pd/alkali metal/Au system) or cadmium and/or the compounds thereof (Pd/alkali metal/Cd system) or barium and/or the compounds thereof (Pd/alkali metal/Ba system) or palladium, alkali metal compounds and mixtures of gold and/or cadmium and/or barium as the catalytically active components on a support, **characterised in that** the support comprises a moulding based on pyrogenically produced mixed oxide having the following physicochemical parameters:
| | |
|---|---|
| External diameter | 0.8-25 mm |
| BET surface area | 5-400 m²/g |
| Pore volume | 0.2-1.8 ml/g |
| Fracture strength | 5-350 N |
| Composition | At least two from the group SiO₂, Al₂O₃, TiO₂ and ZrO₂ in any desired combination but with the exception of SiO₂/Al₂O₃ mixed oxides. |
| Other constituents | < 1 wt.% |
| Bulk density | 250-1500 g/l. |

2. Supported catalyst according to claim 1, **characterised in that** the Pd/K/Au system is used as the active component.

3. Supported catalyst according to claim 1 and/or 2, **characterised in that** the alkali metal compound comprises potassium acetate.

4. Process for the production of the supported catalyst according to one or more of claims 1 to 3 by, in a suitable order, applying the Pd, Au, Cd, Ba metal compounds by impregnation, spraying, vapour deposition, immersion or precipitation, optionally reducing the reducible metal compounds applied onto the support, washing to remove any optionally present chloride content, performing impregnation with alkali metal acetates or alkali metal compounds which are entirely or partially converted into alkali metal acetates under the reaction conditions for the production of vinyl acetate monomer, **characterised in that** the support is a moulding according to claim 1.

5. Process for the production of the supported catalyst according to one or more of claims 1 to 3 by impregnating the support with a basic solution and a solution containing gold and palladium salts, wherein impregnation proceeds simultaneously or in succession, with or without intermediate drying, washing the support to remove any optionally present chloride content and, before or after washing, reducing the insoluble compounds precipitated on the support, drying the resultant catalyst precursor, and performing impregnation with alkali metal acetates or alkali metal compounds which are entirely or partially converted into alkali metal acetates under the reaction conditions for the production of vinyl acetate monomer, **characterised in that** the support is a moulding according to claim 1.

6. Process according to claim 4 or 5, **characterised in that** the alkali metal acetate or the alkali metal compound comprises potassium acetate.

7. Use of a supported catalyst according to one or more of claims 1 to 3 for the production of unsaturated esters from olefins, organic acids and oxygen in the gas phase.

8. Use of a supported catalyst according to claim 7 for the production of vinyl acetate monomer.

## Revendications

1. Catalyseur sur support qui contient sur un support comme composants actifs catalytiquement, du palladium et/ou ses dérivés et des composés de métal alcalin ainsi qu'en supplément de l'or et/ou ses dérivés (système Pd/métal alcalin/Au), ou du cadmium et/ou ses dérivés (système Pd/métal alcalin/Cd), ou du Baryum et/ou ses dérivés (système Pd/métal alcalin/Ba), ou du palladium, des dérivés de métal alcalin et des mélanges à base d'or et/ou de cadmium et/ou de baryum,
**caractérisé en ce qu'**
il s'agit en ce qui concerne le support d'un solide moulé à base d'oxyde mixte produit par voie pyrogénique ayant les caractéristiques physicochimiques suivantes :
| | |
|---|---|
| Diamètre extérieur | 0,8-25 mm |
| Surface BET | 5-400 m²/g |
| Volume des pores | 0,2-1,8 ml/g |
| Résistance à la rupture | 5 à 350 N |
| Composition | au moins deux éléments dans le groupe formé de SiO₂, Al₂O₃, TiO₂, et ZrO₂ dans n'importe quelle combinaison, cependant à l'exception des oxydes mixtes SiO₂-Al₂O₃. |
| Autres constituants | <1 % en poids |
| densité apparente | 250-1500 g/l |

2. Catalyseur sur support conformément à la revendication 1,
**caractérisé en ce qu'**
on utilise comme composants actifs le système Pd/K/Au.

3. Catalyseur sur support conformément à la revendication 1 et/ou 2,
**caractérisé en ce qu'**
il s'agit en ce qui concerne le dérivé de métal alcalin, d'acétate de potassium.

4. Procédé de préparation du catalyseur sur support selon l'une ou plusieurs des revendications 1 à 3, par imprégnation, pulvérisation, métallisation, immersion ou précipitation des dérivés métalliques du Pd, de l'Au, du Cd et du Ba, le cas échéant réduction des dérivés métalliques réductibles appliqués sur le support, lavage pour éliminer des fractions de chlorures éventuellement présents, imprégnation avec des acétates de métal alcalin ou des dérivés de métal alcalin, qui se transforment dans les conditions de la réaction au cours de la production du monomère d'acétate de vinyle, totalement ou partiellement en acétate de métal alcalin, dans une séquence appropriée,
**caractérisé en ce que**
le support est un solide moulé selon la revendication 1.

5. Procédé de préparation du catalyseur sur support selon l'une ou plusieurs des revendications 1 à 3, par imprégnation du support avec une solution basique et une solution contenant un sel d'or et un sel de palladium dans lequel l'imprégnation s'effectue simultanément ou successivement avec ou sans séchage intermédiaire, lavage du support pour l'élimination des fractions de chlorures éventuellement présents, et réduction des composés insolubles précipités sur le support avant ou après le lavage, séchage du stade préliminaire de catalyseur ainsi obtenu, et imprégnation avec des acétates de métal alcalin ou avec des dérivés de métal alcalin, qui se transforment dans les conditions de la réaction au cours de la production de monomère d'acétate de vinyle, totalement ou partiellement en acétate de métal alcalin, dans une séquence appropriée,
**caractérisé en ce que**
le support est un solide moulé selon la revendication 1.

6. Procédé selon la revendication 4 ou la revendication 5,
**caractérisé en ce qu'**
il s'agit en ce qui concerne l'acétate de métal alcalin ou de dérivés de métal alcalin, d'acétate de potassium.

7. Utilisation d'un catalyseur sur support selon l'une ou plusieurs des revendications 1 à 3, pour la production d'esters non saturés à partir d'oléfines, d'acides organiques et d'oxygène en phase gazeuse.

8. Utilisation d'un catalyseur sur support conformément à la revendication 7, pour la production de monomère d'acétate de vinyle.
